# EUROPEAN PATENT SPECIFICATION

(11) **EP 2 166 990 B1**
(45) Date of publication and mention of the grant of the patent: **03.10.2018**
(21) Application number: 08795325.3
(22) Date of filing: 13.08.2008
(51) Int. Cl.: A61F 2/40, A61F 2/46, A61B 17/56, A61M 29/02, A61F 2/30

(54) **TISSUE POSITIONING DEVICE**
GEWEBE-POSITIONIERUNGSVORRICHTUNG
DISPOSITIF DE POSITIONNEMENT DE TISSU

(30) Priority: 13.08.2007 US 964703 P
(43) Date of publication of application: 31.03.2010
(60) Divisional application: 14157065.5
(73) Proprietor: MicroAire Surgical Instruments, LLC, Charlottesville, VA 22911 (US)
(72) Inventor: MORRIS, Stephen, Chico, CA 95928 (US); WEISEL, Thomas, Ventura, CA 93003 (US); PISARNWONGS, Roger, Valencia, CA 91354 (US)
(74) Representative: Somervell, Thomas Richard
(86) International application number: PCT/US2008/009729
(87) International publication number: WO 2009/023250

(56) References cited:
- WO-A-98/20939
- FR-A- 2 803 191
- US-A1- 2004 092 948
- US-A1- 2004 267 277
- US-A1- 2005 229 433
- US-A1- 2007 078 477

## Description

### RELATED APPLICATIONS

This application claims the benefit of provisional patent application number 60/964,703 to S. Morris et al., filed August 13, 2007.

### BACKGROUND OF THE INVENTION

### Field of the Invention

This invention relates generally to tissue positioning devices, and more particularly to devices for repositioning tissues that have been displaced due to injury or illness.

### Description of the Related Art

Medical practitioners often see patients with ailments caused by soft or hard tissue displacements relative to the surrounding anatomy. Much effort is placed into repositioning the tissue and keeping it in the correct location. A common example is a broken bone, where the doctor repositions the bone and restricts its movement via a cast until the bones are healed.

A variety of different devices are used to reposition tissue, such as casts and splints, screws and plates, and spacers such as those used in the spine. These devices work fine for their indicated uses, but may be inadequate for a heavily articulatable joint such as the shoulder.

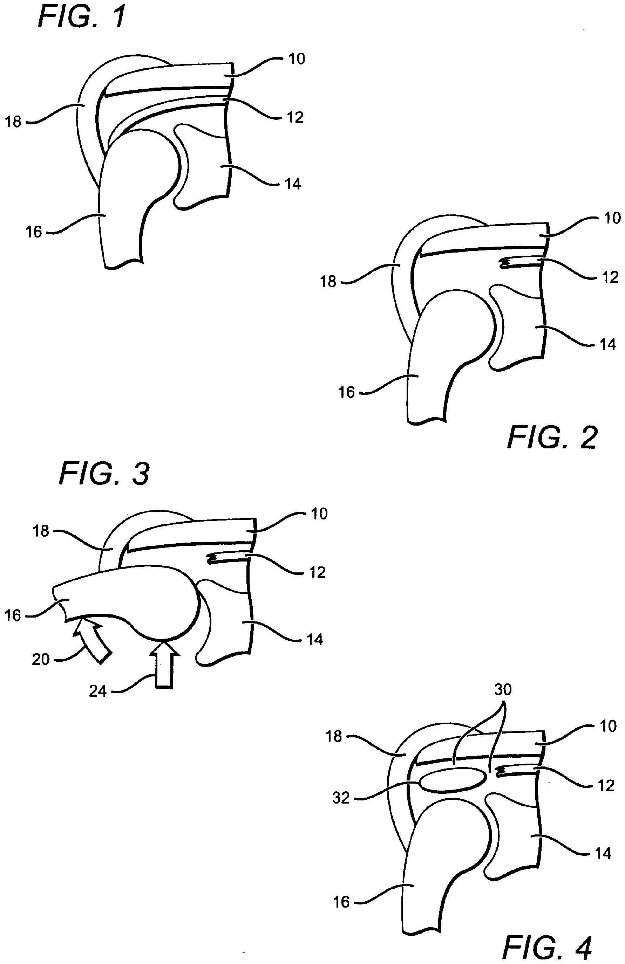

FIGs. 1-3 describe a situation that can arise in the shoulder. FIG. 1 depicts a simplified cross-sectional view of the shoulder joint. The acromium 10, rotator cuff tendons 12, the glenoid 14, the humerus 16, and the deltoid muscle 18 are shown. In a healthy shoulder, the rotator cuff tendons 12 hold the head of the humerus 16 in the cup of the glenoid 14, so that even if muscles such as the deltoid 18 pull on the humerus, its head remains in the cup of the glenoid.

However, in FIG. 2, rotator cuff tendons 12 have been drastically reduced, such that they can no longer hold the head of the humerus 16 in the glenoid 14 as deltoid muscle 18 pulls on the humerus. FIG. 3 shows that as the person raises his arm, he utilizes his deltoid muscle 18, which rotates the humerus 16 in an upward direction 20. Since the rotator cuff tendons 12 are not holding humerus 16 in place, its head tends to lift (24) out of the cup of glenoid 14, creating a very painful movement for the patient and impairing the capacity for activities above shoulder level.

The most common methods of treatment for this condition are lengthy physical therapy, partial or total shoulder replacement surgery, reverse total shoulder arthoplasty, or doing nothing, in which case the patient continues to experience pain and loss of strength.

**Patent application** US 2007/0078477 A1 **discloses an anatomical spacer for placement in an anatomical location, and a method to deploy the anatomical spacer. The spacer contains an inflatable bladder with inflation port, and has opposing sufaces facing each other, with a membrane extending between them.**

### SUMMARY OF THE INVENTION

**According to the present invention there is provided a tissue positioning device as set forth in claim 1.** The present invention is directed to a tissue positioning device that addresses the issues noted above, in that it intervenes with minimal inconvenience to the patient, while allowing the performance of normal activities with reduced pain and increased strength.

The device comprises a biocompatible member having a size and shape suitable for placement within a space adjacent to a tissue to be positioned; the tissue, which may be hard or soft, forms a portion of an articulatable joint. Once placed within the space, the member acts to maintain the tissue in a desired position. The member may be a spacer having a defined shape, or a bladder capable of receiving and being at least partially expanded by a filler material.

When configured as a spacer, the member may be rigid or flexible, and has a size and shape suitable for placement within a space adjacent to a particular tissue. The spacer can be made from any of a number of different materials, such as silicone rubber and/or ultra high molecular weight polyethylene (UHMWPE), as well as super-elastic or shape-memory materials capable of being compressed for insertion into the space, and then reverting to a preformed shape.

When the member is a bladder, the bladder has an associated deflated state and is capable of receiving and being at least partially expanded by a filler material; a valve is provided by which a filler material can be delivered. The bladder is capable of insertion into the space adjacent to the tissue when in its deflated state, and acts to maintain the tissue in a desired position when at least partially expanded by the delivery of filler material.

The bladder may be arranged such that it continues to expand as long as additional filler material is delivered, or to only expand up to a predetermined limit. The bladder can made in whole or part from a variety of materials, including, for example, silicone rubber, cross-linked polyethylene (PE), polyester (PET), metal, woven Kevlar, UHMWPE, stainless steel, and Nitinol. The filler material can be any of a number of substances, including liquids, gases, a curable liquid such as bone cement or urethane foam, or even a spring.

The present tissue positioning device may include an attachment means by which the member can be secured to one or more tissues such that it is maintained in a desired spatial location. Suitable attachment means include a tab affixed to or molded as part of the member, with the tab having a suture embedded within it or containing a hole through which a suture may be threaded. The bladder might also be arranged to accommodate one or more attachment means such as bone anchors that can be inserted into adjacent hard or soft tissue.

These and other features, aspects, and advantages of the present invention will become better understood with reference to the following description and claims.

### BRIEF DESCRIPTION OF THE DRAWINGS

FIGs. 1-3 are cross-sectional views of a shoulder joint which illustrate a typical tissue displacement condition which may be addressed by the present invention.
FIG. 4 is a cross-sectional view of a shoulder joint which illustrates the placement of a bladder-type tissue positioning device within a space adjacent to a tissue to be positioned.
FIGs. 5 and 6 are cross-sectional views of a shoulder joint which illustrate how expanding the bladder of FIG. 4 acts to maintain the tissue in a desired position.
FIGs. 7a and 7b are cross-sectional views of a bladder and valve.
FIG. 8 is a cross-sectional view of a bladder which uses a spring as a filler material.
FIG. 9 is a cross-sectional view of a bladder illustrating several possible attachment means.
FIG. 10 is a cross-sectional view of a bladder having a non-uniform thickness.
FIG. 11 is a cross-sectional view of a bladder in which a portion of the bladder comprises a reinforced material.
FIG. 12 is a cross-sectional view of a bladder to which a secondary plate has been affixed.
FIG. 13 is a cross-sectional view of a bladder composed of two or more different materials.
FIG. 14 is a cross-sectional views of a shoulder joint which illustrates the placement of a spacer-type tissue positioning device within a space adjacent to a tissue to be positioned.
FIGs. 15a-15c are plan and sectional views of one possible embodiment of a spacer-type tissue positioning device.

### DETAILED DESCRIPTION OF THE INVENTION

The present tissue positioning device comprises a biocompatible member having a size and shape suitable for placement within a space adjacent to a tissue to be positioned. The tissue, which may be hard (such as bone, etc.) or soft (such as muscle or tendon), forms a portion of an articulatable joint. Once deployed within the space, the member acts to maintain the tissue in a desired position. The use of the device within a shoulder joint is described below, but the device may also be used to address injuries within other articulating joints.

A simplified cross-sectional view of a shoulder joint which includes a tissue positioning device in accordance with the present invention is shown in FIG. 4. The acromium 10, rotator cuff tendons 12, the glenoid 14, the humerus 16, and the deltoid muscle 18 are shown. In a healthy shoulder, the rotator cuff tendons 12 hold the head of humerus 16 in the cup of glenoid 14, so that even if muscles such as deltoid 18 pull on the humerus, its head remains in the cup of the glenoid.

Here, however, due to illness or injury, rotator cuff tendons 12 have been significantly reduced such that they no longer act to hold the head of humerus 16 in the cup of glenoid 14. As noted above, this results in the head tending to lift out of the cup of the glenoid, creating a very painful movement for the patient and limiting function above shoulder level.

The tissue positioning device is placed within a space adjacent to a tissue to be positioned. Here, the shortened rotator cuff tendons 12 leave an open space 30 adjacent to the tissue to be positioned, which in this example is humerus 16. The device 32 is placed within space 30, between acromium 10, humerus 16 and deltoid 18, preferably via an arthroscopic port (skin or cannula) with the aid of a scope, or through a larger skin incision with direct visualization.

Device 32 may be in the form of a spacer which has a generally defined shape when in place within the space, or a bladder which can be at least partially expanded when in place within the space. A bladder-type tissue positioning device is discussed first.

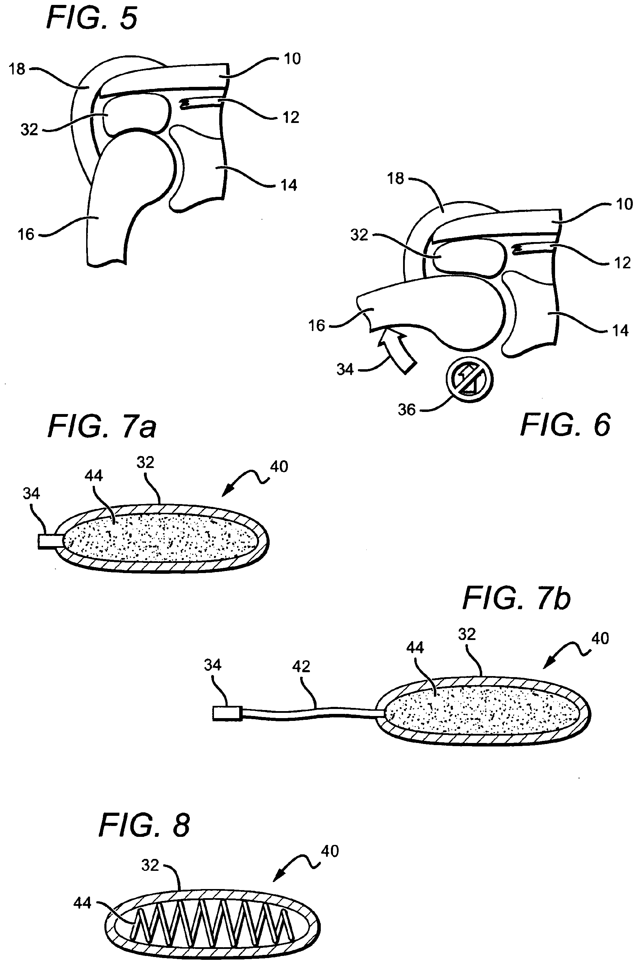

The method by which a bladder-type device might be used is illustrated in FIGs. 5 and 6. In FIG. 5, bladder 32 is shown in an expanded state, with the patient's arm hanging straight down. In FIG. 6, the patient lifts (34) his arm and humerus 16 with the aid of deltoid 18. Previously, this motion tended to cause the head of humerus 16 to lift out of the cup of glenoid 14. Now, however, there is no upward translation (36), because expanded bladder 32 places a downward force on the head of humerus 16, with the aid of backing from acromium 10. This bladder can also be attached to one or multiple structures such as the glenoid or acromium, as discussed below.

Depending on the specific application, bladder 32 might be made to be expandable, expandable up to a predetermined limit, or not expandable at all if its fit within the space is proper without expansion. In the latter case, the bladder becomes more like a defined-sized spacer. One advantage of using a bladder which is expandable is that the bladder can be placed in the shoulder via a small portal, and then expanded to a much larger size once positioned within the joint - thus minimizing patient trauma due to inserting a large fixed-size device.

Various details for possible bladder-type embodiments are illustrated in the cross-sectional views shown in FIGs. 7 to 13. In FIG. 7a, tissue positioning device 40 includes a bladder 32 and a valve 34 by which a filler material can be delivered into the bladder. Many different types of valves could be employed, including, for example, a needle-piercable rubber type or a spring-loaded ball type. The valve may be integral to the bladder, as shown in FIG. 7a, or tethered to the bladder via a communicating tube 42 as illustrated in FIG. 7b. The valve preferably enables bladder 32 to be easily filled, and then allow no leakage of the filler material. The valve may be a one-way valve which only allows filler material to be added to the bladder, or a two-way valve which would also allow for removal of the material from inside bladder 32, in order to deflate the bladder as required. The valve is preferably positioned just below the skin such that it can be easily accessed using, for example, a syringe.

The bladder cross-section shown in FIGs. 7a and 7b is slightly ovoid, but the shape and size can be almost anything that properly matches the anatomy in need of repair and which fits within the available space.

Bladder 32 is expanded by way of a filler material 44 delivered via valve 34. The filler material can be one substance or a combination of many different substances. The filler material's properties must allow for adequate expansion of bladder 32, and must adequately hold the head of humerus 16 in place during manipulation. Examples of suitable filler materials include air or any other gas, silicone, saline or any other liquid, a gel such as hyaluronic acid, and cured (reacting) substances such as bone cement or urethane foams.

As shown in FIG. 8, filler material 44 might also take the form of a spring. Such a spring can be compressed while the bladder is being positioned, and allowed to expand once in place. The spring can be made of any appropriate metal or plastic material, such as Nitinol. The spring might also be a super-elastic or shape memory material capable of being compressed for insertion into the space, and then reverting to a preformed shape. For example, the spring could be made from a shape memory material that is temperature activated so that it expands once the device warms to body temperature. The spring could be made to be expandable in one, two or three dimensions, as needed.

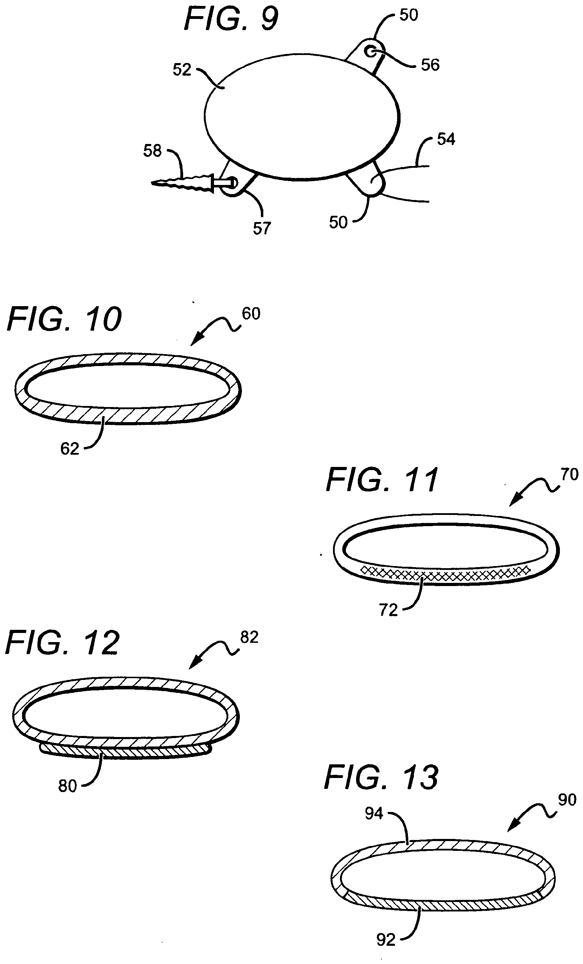

The present tissue positioning device may include an attachment means by which the member can be secured to one or more anatomical structures such that it is maintained in a desired spatial location. Various possible attachment means are illustrated in FIG. 9. For example, tabs 50 can be affixed to or molded as part of member 52, which can be either a bladder or defined-shape spacer. The tab may have a suture 54 embedded within it, or contain a hole 56 through which a suture may be threaded; the suture could then be tied to, for example, a bone anchor, or directly to other hard or soft tissue (not shown) as appropriate.

Another possibility is to provide a tab 57 to which an anchor device 58 such as a bone anchor or tack has been affixed, or through which an anchor device can be routed. The anchor device or devices would then be attached to appropriate hard or soft tissue as needed.

Various possibilities related to the composition of the bladder are addressed in FIGs. 10-13. In the case of the shoulder joint, there may be concern with wear on the side of the bladder that contacts the moving humeral head, or with retaining the general bladder structure. As such, a bladder with a uniform wall thickness and composition may have to be modified. One possible modification is illustrated in the cross-sectional view of bladder 60 in FIG. 10. Here, the side of the bladder which rubs against the humeral head will be thickened (62) to create a more durable wall.

Another possibility is shown in FIG. 11, in which at least a portion of the bladder 70 comprises a reinforced material 72, to reduce the degradation of the bladder due to its contact with the tissue to be positioned. A reinforced material could also be used to strengthen the attachment tabs referred to above. This material could be a non-easily abraded material such as a woven Kevlar, UHMWPE, stainless steel, Nitinol, etc. The reinforced material might also be in the form of a mesh affixed to the side of bladder 70, which contacts the tissue to be positioned and thereby protects the bladder.

In FIG. 12, a secondary plate 80 is affixed to the bottom of a bladder 82 to act as a buffer against abrasion. The plate's material, as with any of the materials that contact the tissue to be positioned, may have a lubricious quality such as UHMWPE. A thin layer of Nitinol which can be unfurled in the joint may also work as a buffer layer.

Another possibility is shown in FIG. 13, in which the member 90 is made from two or more different materials. For instance, the portion 92 that contacts the humeral head may be a hard lubricious plastic, with an expandable rubber bladder portion 94 overmolded onto portion 92 which can be properly filled to occupy the space in question. Portion 92 may also contain a molded-in lubricant, such as silicone oil, to help minimize wear. One consideration in choosing the material(s) for this embodiment, as with all previously discussed embodiments, is the need to minimize wear on the tissue being positioned.

The material for the bladder can be flexible (e.g., silicone rubber) or relatively non-expanding (e.g., cross-linked PE). The bladder could also be made of metal, in the form of a bellows, for example, which can be inflated to the desired size. Other possible bladder materials include, but are not limited to, polyester (PET), metal, woven Kevlar, ultra high molecular weight polyethylene (UHMWPE), stainless steel, and Nitinol.

A tissue positioning device in accordance with the present invention may be either a bladder-type as described above, or a spacer-type, in which the member has a generally defined shape when in place within the space and acts to maintain a particular tissue in a desired position.

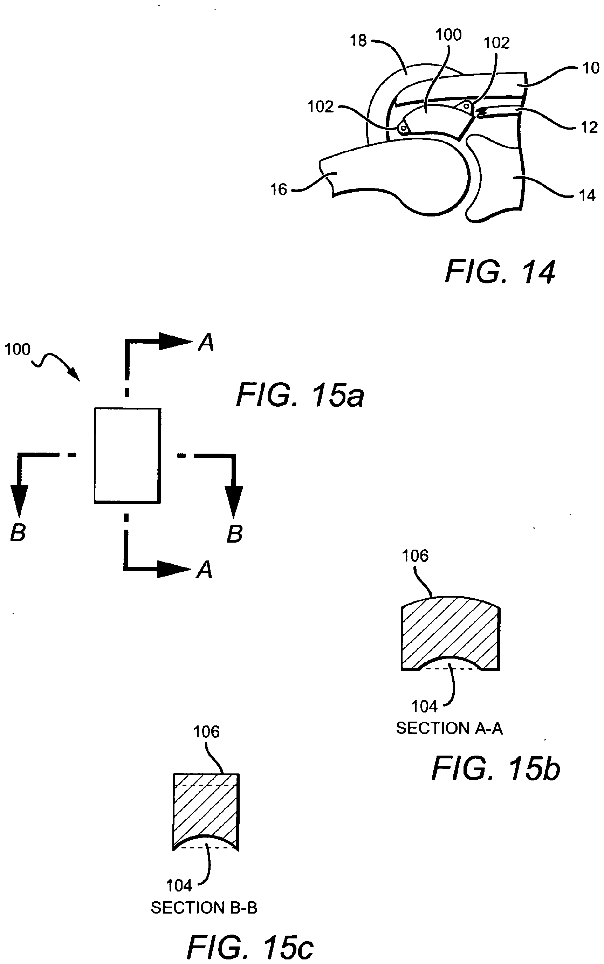

A cross-sectional view of a spacer-type tissue positioning device as might be used within a shoulder joint is shown in FIG. 14. As before, device 100 is placed within a space created by the degradation of rotator cuff tendons 12. Once in place, device 100 acts to maintain a particular tissue in a desired position - here, spacer 100 acts to maintain the head of humerus 16 in the cup of glenoid 14.

Device 100 can be rigid or flexible, or some portions may be rigid and others flexible, as needed. The device can be made from a wide variety of materials, such as silicone rubber and/or UHMWPE. A molded-in lubricant might also be used, to reduce friction between the spacer and the tissue being positioned. The device might also be made from a composite material, such that different portions of the member have different physical characteristics - for example, the spacer might be designed such that the surface that contacts the humerus is relatively hard, while the surface that contacts the acromium is relatively soft. Some or all of a spacer-type tissue positioning device might also be made from a super-elastic or shape-memory material capable of being compressed for insertion into the space, and then reverting to a preformed shape.

As with bladder-type embodiments, the spacer may include one or more attachment means by which it can be secured to nearby tissues or anchor devices. For example, tabs 102 can be affixed to or molded as part of member 100; the tab could have a suture embedded within it, or contain a hole through which a suture may be threaded. An anchor device (not shown) such as a bone anchor or tack might also be affixed to or routed through one or more tabs. The sutures or anchor devices would then be attached to appropriate hard or soft tissue as needed.

The device is designed to have a size and shape suitable for placement within a given space and for positioning a particular tissue. For example, a spacer-type device designed to maintain the head of humerus 16 in the cup of glenoid 14 may have a cup-like recess within the surface of the spacer that contacts the humerus. This is illustrated in FIGs. 15a (plan view), 15b (cut along section line A-A), and 15c (cut along section line B-B); here, according to the invention, the surface which contacts the acromium 10 is generally rounded, while the opposite surface includes a recessed space 104 to assist in maintaining the humeral head in the proper position.

In practice, it may be necessary to insert one or more trial devices, to make sure that the proper fit is achieved or the proper bladder or spacer is used. A measuring forceps might be employed to obtain the size of the space in which the device is to be placed, in order to choose the correct device size.

The embodiments of the invention described herein are exemplary and numerous modifications, variations and rearrangements can be readily envisioned to achieve substantially equivalent results, all of which are intended to be embraced within the scope of the invention as defined in the appended claims.

## Claims

1. A tissue positioning device (40), comprising:
a biocompatible member (52) having a size and shape suitable for placement within a space (30) adjacent to a tissue to be positioned, said tissue forming a portion of an articulatable joint, wherein said space is between the acromium (10), deltoid muscle (18), and humerus (16), such that, while placed within said space, said member acts to maintain the head of said humerus within the cup of the glenoid (14), and said biocompatible member having a rounded first surface contacting a surface of the acromium (10) and a second surface having a recessed space (104), the second surface contacting the humeral head of the humerus (16).

2. The device (40) of claim 1, wherein said member (52) is a spacer which has a defined shape when acting to maintain the head of said humerus (16) within the cup of the glenoid (14).

3. The device (40) of claim 2, wherein at least a portion of said spacer comprises a super-elastic or shape-memory material capable of being compressed for insertion into said space (30), and then reverting to a preformed shape.

4. The device (40) of claim 2, wherein said member (52) comprises a composite material such that different portions of said member have different physical characteristics.

5. The device (40) of claim 1, wherein said member (52) comprises:
a bladder (32, 70) having an associated deflated state and which is capable of receiving and being at least partially expanded by a filler material (44); and
a valve (34) by which a filler material can be delivered into said bladder;
such that said bladder is capable of insertion into said space (30) when in said deflated state and acts to maintain the head of said humerus (16) within the cup of the glenoid (14) when at least partially expanded by the delivery of filler material via said valve.

6. The device (40) of claim 5, wherein said filler material (44) is selected from a group consisting of a liquid, a gas, and a curable liquid.

7. The device (40) of claim 1, wherein at least a portion of said device comprises a material selected from a group consisting of silicone rubber, cross-linked polyethylene (PE), polyester (PET), metal, woven Kevlar, ultra high molecular weight polyethylene (UHMWPE), stainless steel, and Nitinol.

8. The device (40) of claim 1, further comprising an attachment means by which said member (52) can be secured to one or more tissues such that it is maintained in a desired spatial location.

9. The device (40) of claim 5, wherein at least a portion of said bladder (32, 70) comprises a reinforced material (72) to reduce the degradation of said bladder due to its contact with said tissue to be positioned.

10. The device (40) of claim 9, wherein said reinforced material (72) is in the form of a mesh.

11. The device (40) of claim 5, further comprising a secondary plate (80) affixed to said bladder (32, 70) such that said plate provides a buffer between said bladder and said tissue to be positioned.

12. The device (40) of claim 5, further comprising a communicating tube (42) which tethers said valve (34) to said bladder (32, 70).

13. The device (40) of claim 5, wherein said filler material (44) is a spring which is expandable in one, two, or three dimensions.

## Patentansprüche

1. Vorrichtung zur Positionierung von Gewebe (40), umfassend:
ein biokompatibles Element (52), das derart bemessen und geformt ist, dass es für eine Platzierung innerhalb eines Raums (30), der an ein zu positionierendes Gewebe angrenzt, geeignet ist, wobei das Gewebe einen Teil eines schwenkbaren Gelenks bildet, wobei der Raum zwischen dem Akromion (10), dem Deltamuskel (18) und dem Humerus (16) liegt, sodass das Element, wenn es innerhalb des Raums platziert ist, bewirkt, dass der Humeruskopf innerhalb der Schulterpfanne (14) gehalten wird, und wobei das biokompatible Element Folgendes aufweist: eine gerundete erste Oberfläche, welche eine Oberfläche des Akromions (10) kontaktiert und eine zweite Oberfläche, welche einen ausgesparten Raum (104) aufweist, wobei die zweite Oberfläche den Humeruskopf des Humerus (16) kontaktiert.

2. Vorrichtung (40) nach Anspruch 1, wobei das Element (52) ein Abstandshalter mit einer definierten Form ist, wenn er bewirkt, dass der Kopf des Humerus (16) innerhalb der Schulterpfanne (14) gehalten wird.

3. Vorrichtung (40) nach Anspruch 2, wobei mindestens ein Teil des Abstandshalters einen superelastischen Werkstoff oder einen Werkstoff mit Formgedächtnis umfasst, der für eine Einsetzung in den Raum (30) komprimiert werden kann und danach wieder eine vorgeformte Gestalt annimmt.

4. Vorrichtung (40) nach Anspruch 2, wobei das Element (52) einen Verbundwerkstoff umfasst, sodass verschiedene Teile des Elements unterschiedliche physische Merkmale aufweisen.

5. Vorrichtung (40) nach Anspruch 1, wobei das Element (52) Folgendes umfasst:
einen Ballon (32, 70), der einen dazugehörigen Leerzustand aufweist und dazu in der Lage ist, einen Füllstoff (44) aufzunehmen und mindestens teilweise von diesem ausgedehnt zu werden; und
ein Ventil (34), durch das ein Füllstoff derart in den Ballon eingefüllt werden kann, dass der Ballon, wenn er im Leerzustand ist, in den Raum (30) eingesetzt werden kann und, wenn er mindestens teilweise durch das Einfüllen von Füllstoff über das Ventil ausgedehnt worden ist, bewirkt, dass der Kopf des Humerus (16) innerhalb der Schulterpfanne (14) gehalten wird.

6. Vorrichtung (40) nach Anspruch 5, wobei der Füllstoff (44) aus einer Gruppe ausgewählt ist, die aus einer Flüssigkeit, einem Gas und einer härtbaren Flüssigkeit besteht.

7. Vorrichtung (40) nach Anspruch 1, wobei mindestens ein Teil der Vorrichtung einen Werkstoff umfasst, der aus einer Gruppe ausgewählt ist, die aus Silikonkautschuk, vernetztem Polyethylen (PE), Polyester (PET), Metall, gewebtem Kevlar, ultrahochmolekularem Polyethylen (UHMWPE), Edelstahl und Nitinol besteht.

8. Vorrichtung (40) nach Anspruch 1, die ferner ein Befestigungsmittel umfasst, mit dem das Element (52) an einem oder mehreren Geweben derart fixiert werden kann, dass es an einem gewünschten räumlichen Standort gehalten wird.

9. Vorrichtung (40) nach Anspruch 5, wobei mindestens ein Teil des Ballons (32, 70) einen verstärkten Werkstoff (72) umfasst, um den Verschleiß des Ballons aufgrund seines Kontakts mit dem zu positionierenden Gewebe zu mindern.

10. Vorrichtung (40) nach Anspruch 9, wobei der verstärkte Werkstoff (72) eine Netzform aufweist.

11. Vorrichtung (40) nach Anspruch 5, die ferner eine sekundäre Scheibe (80) umfasst, die am Ballon (32, 70) angebracht ist, sodass die Scheibe einen Puffer zwischen dem Ballon und dem zu positionierenden Gewebe bildet.

12. Vorrichtung (40) nach Anspruch 5, die ferner ein Verbindungsröhrchen (42) umfasst, das das Ventil (34) mit dem Ballon (32, 70) verbindet.

13. Vorrichtung (40) nach Anspruch 5, wobei der Füllstoff (44) eine Feder ist, die in einer, zwei oder drei Dimensionen dehnbar ist.

## Revendications

1. Dispositif de positionnement de tissu (40) comprenant :
un membre biocompatible (52) ayant une taille et une forme adaptées pour un placement dans un espace (30) adjacent à un tissu devant être positionné, ledit tissu formant une partie d'un joint articulé ; dans lequel ledit espace est situé entre l'acromion (10), le muscle deltoïde (18) et l'humérus (16) de telle sorte que lors de son positionnement dans ledit espace, ledit membre agit pour maintenir la tête dudit humérus dans la cavité glénoïde (14), et ledit membre biocompatible ayant une première surface arrondie en contact avec une surface de l'acromion (10) et une deuxième surface ayant un espace évidé (104), la deuxième surface étant en contact avec la tête de l'humérus (16).

2. Dispositif (40) selon la revendication 1, dans lequel ledit membre (52) est une entretoise ayant une forme définie lorsqu'il agit pour maintenir la tête dudit humérus (16) dans la cavité glénoïde (14).

3. Dispositif (40) selon la revendication 2, dans lequel au moins une partie de ladite entretoise comprend un matériau super-élastique ou à mémoire de forme pouvant être comprimé pour une insertion dans ledit espace (30), pour ensuite revenir à une forme préformée.

4. Dispositif (40) selon la revendication 2, dans lequel ledit membre (52) comprend un matériau composite de telle sorte que différentes parties dudit membre présentent des caractéristiques physiques différentes.

5. Dispositif (40) selon la revendication 1, dans lequel ledit membre (52) comprend :
une vessie (32, 70) présentant un état dégonflé associé et étant capable de recevoir un matériau de remplissage (44) et d'être au moins partiellement dilatée par celui-ci ;
une valve (34) par laquelle un matériau de remplissage peut être délivré dans ladite vessie,
de telle sorte que ladite vessie peut être insérée dans ledit espace (30) lorsqu'elle se trouve dans ledit état dégonflé et agit de manière à maintenir la tête dudit humérus (16) dans la cavité glénoïde (14) lorsqu'elle est au moins partiellement dilatée par l'introduction du matériau de remplissage par ladite valve, et

6. Dispositif (40) selon la revendication 5, dans lequel ledit matériau de remplissage (44) est sélectionné dans le groupe constitué d'un liquide, d'un gaz et d'un liquide durcissable.

7. Dispositif (40) selon la revendication 1, dans lequel au moins une partie dudit dispositif comprend un matériau sélectionné dans le groupe constitué de caoutchouc de silicone, de polyéthylène réticulé (PE), de polyester (PET), de métal, de Kevlar tissé, de polyéthylène de poids moléculaire ultra élevé (UHMWPE), d'acier inoxydable et de nitinol.

8. Dispositif (40) selon la revendication 1, comprenant en outre un moyen de fixation par lequel ledit membre (52) peut être fixé à un ou plusieurs tissus de telle sorte qu'il est maintenu dans une position spatiale désirée.

9. Dispositif (40) selon la revendication 5, dans lequel au moins une partie de ladite vessie (32, 70) comprend un matériau renforcé (72), afin de réduire la dégradation de ladite vessie en raison de son contact avec ledit tissu devant être positionné.

10. Dispositif (40) selon la revendication 9, dans lequel ledit matériau renforcé (72) présente la forme d'une maille.

11. Dispositif (40) selon la revendication 5, comprenant en outre un disque secondaire (80) attaché à ladite vessie (32, 70) de telle sorte que ledit disque fournit un tampon entre ladite vessie et ledit tissu devant être positionné.

12. Dispositif (40) selon la revendication 5, comprenant en outre un tube de communication (42) qui relie ladite valve (34) à ladite vessie (32, 70).

13. Dispositif (40) selon la revendication 5, dans lequel ledit matériau de remplissage (44) est un ressort qui peut être étiré dans une, deux ou trois dimensions.
